# EUROPEAN PATENT SPECIFICATION

(11) **EP 2 276 835 B1**
(45) Date of publication and mention of the grant of the patent: **15.08.2012**
(21) Application number: 09745571.1
(22) Date of filing: 08.05.2009
(51) Int. Cl.: C12N 9/16

(54) **Process for the preparation of a phospholipase**
Verfahren zur Herstellung einer Phospholipase
Procédé de préparation d'une phospholipase

(30) Priority: 15.05.2008 RU 2008119295
(43) Date of publication of application: 26.01.2011
(73) Proprietor: LODERS CROKLAAN B.V., 1521 AZ Wormerveer (NL)
(72) Inventor: TARAN, Viktoria, NL-2628 CV Delft (NL); DRYGIN, Yuri, Fedorovich, Moscow 119991 (RU); ZUEVA, Vera, Sergeevna, 117997 (RU); GALIULLINA, Raisa, Anvarovna, Moscow 117209 (RU)
(74) Representative: Stevens, Ian Edward
(86) International application number: PCT/EP2009/003454
(87) International publication number: WO 2009/138237

(56) References cited:
- SHUMILINA E V ET AL: "Influence of coprecipitation and interactions with anionic surfactants on the activity of plant phospholipase D" BIOCHEMISTRY, CONSULTANTS BUREAU, NEW YORK, NY, US, vol. 64, no. 8, 1 August 1999 (1999-08-01), pages 883-889, XP008108758 ISSN: 0006-2979
- SHUMILINA E V ET AL: "Effects of surfactants on the activity of phospholipase D" MEMBRANE AND CELL BIOLOGY, HARWOOD ACADEMIC PUBLISHERS, CHUR, CH, vol. 12, no. 4, 1 January 1998 (1998-01-01), pages 513-520, XP008108730 ISSN: 1023-6597
- RENATE ULBRICH-HOFMANN ET AL: "Phospholipase D and its application in biocatalysis" BIOTECHNOLOGY LETTERS, KLUWER ACADEMIC PUBLISHERS, DO, vol. 27, no. 8, 1 April 2005 (2005-04-01), pages 535-544, XP019231148 ISSN: 1573-6776
- SHARMA SHWETA ET AL: "One step purification of peanut phospholipase D by precipitation with alginate" BIOSEPARATION, vol. 9, no. 2, 2000, pages 93-98, XP002538577 ISSN: 0923-179X cited in the application
- TEOTIA S ET AL: "Purification of phospholipase D by two-phase affinity extraction" JOURNAL OF CHROMATOGRAPHY, ELSEVIER SCIENCE PUBLISHERS B.V. AMSTERDAM, NL, vol. 1025, no. 2, 6 February 2004 (2004-02-06), pages 297-301, XP004482107 ISSN: 0021-9673
- HELLER M ET AL: "PHOSPHOLIPASE D IN PEANUT SEEDS" BULLETIN DE LA SOCIETE DE CHIMIE BIOLOGIGUE, MASSON, PARIS, FR, vol. 50, no. 9, 1 January 1968 (1968-01-01), pages 1395-1408, XP008108766 ISSN: 0037-9042
- YOZO NAKAZAWA ET AL: "Purification, Characterization and Cloning of Phospholipase D from Peanut Seeds" JOURNAL OF PROTEIN CHEMISTRY, KLUWER ACADEMIC PUBLISHERS-PLENUM PUBLISHERS, NE, vol. 25, no. 3, 16 May 2006 (2006-05-16), pages 212-223, XP019401231 ISSN: 1573-4943
- LEE ET AL: "Phospholipase D of rice bran. I. Purification and characterization" PLANT SCIENCE, ELSEVIER IRELAND LTD, IE, vol. 59, no. 1, 1 January 1989 (1989-01-01), pages 25-33, XP023471848 ISSN: 0168-9452 [retrieved on 1989-01-01]
- LAMBRECHT R ET AL: "A FACILE PURIFICATION PROCEDURE OF PHOSPHOLIPASE D FROM CABBAGE AND ITS CHARACTERIZATION" BIOLOGICAL CHEMISTRY HOPPE-SEYLER, WALTER DE GRUYTER, BERLIN, DE, vol. 373, no. 2, 1 February 1992 (1992-02-01), pages 81-88, XP008108711 ISSN: 0177-3593
- YOSHIO OKAHATA ET AL: "SIMPLE TRANSPHOSPHATIDYLATION OF PHOSPHOLIPIDS CATALYSED BY A LIPID-COATED PHOSPHOLIPASE D IN ORGANIC SOLVENTS" JOURNAL OF THE CHEMICAL SOCIETY, PERKIN TRANSACTIONS 1, CHEMICAL SOCIETY. LETCHWORTH, GB, no. 7, 7 April 1995 (1995-04-07), pages 919-925, XP000604732 ISSN: 0300-922X
- DE MARIA L ET AL: "Phospholipases and their industrial applications" APPLIED MICROBIOLOGY AND BIOTECHNOLOGY, SPRINGER, BERLIN, DE, vol. 74, no. 2, 13 January 2007 (2007-01-13), pages 290-300, XP019538660 ISSN: 1432-0614

## Description

The present invention relates to a process for the preparation of a phospholipase D from carrot.

Phospholipase D from plants and microorganisms is used as a biocatalyst to convert phospholipids into fatty acids and other lipophilic substances. In particular, phospholipase D exhibits both transphosphatidylation and hydrolytic activities for various phospholipids. The transphosphatidylation activity is particularly useful for converting phosphatidylcholine into other phospholipids.

EP-A-1048738 describes a process for the preparation of phosphatidylserine by reacting a phospholipid with serine in an aqueous dispersion in the presence of phospholipase D and calcium salts.

US 2004/0235119 discloses a method for the production of phospholipids involving the use of a phospholipase D.

WO 00/77183 relates to the enzymatic preparation of phospholipids in aqueous media.

Methods of isolating phospholipase D from plants and microorganisms are known. For example, Sharma et al, (2000), Bioseparation., vol. 9, pages 93-98 discloses the purification of peanut phospholipase D by precipitation with alginate. The purification consists of the co-precipitation of phospholipase D with alginate upon addition of 0.06 M Ca²⁺. The enzyme is eluted from the polymer using 0.2 M sodium chloride.

The listing or discussion of an apparently prior-published document in this specification should not necessarily be taken as an acknowledgement that the document is part of the state of the art or is common general knowledge.

There remains a need, however, for a process for preparing a phospholipase D of vegetable or nut origin that can be operated economically and efficiently.

According to the invention, there is provided a process for the preparation of a phospholipase D from carrot comprising the following steps:
(i) providing a liquid carrot extract;
(ii) contacting the liquid extract of (I) with a clarifying agent comprising one or more metal ions to produce a suspension comprising a clarified liquid extract and a precipitate:
(iii) combining:
   the clarified liquid extract of (ii) with a precipitating agent comprising at least one surfactant in admixture with phospholipids and optionally mono-, di- or triglycerides or a mixture thereof to produce a suspension comprising a supernatant and a precipitate; and
(iv) separating and collecting the precipitate from the suspension produced in
(iii) wherein step (ii) is carried out at a pH of from 7 to 9 and wherein the process comprises separating and collecting the clarified liquid extract from the suspension produced in step (ii).

Further embodiments are set out in Claims 2 to 13.

The liquid carrot extract for use in the present invention is preferably provided in the form of a juice, sap or similar liquid preparation. The liquid extract may not be fully liquid and may comprise some solids. Typically, the carrot is cooled to a temperature below room temperature (i.e., below 25°C), such as at a temperature in the range of from 1 to 10°C or from 2 to 7°C, washed and chopped. The juice or sap is then extracted from the chopped preparation by methods known in the art, for example by using a domestic or commercial juice extractor. In some cases a proportion of the original phospholipase D content of the source carrot will be left in the solid wastes after the initial removal of the juice or sap. Optionally, additional phospholipase D may be extracted from these solid wastes by the addition of water and re-processing through the juice extractor. The person skilled in the art will appreciate that the liquid extract obtained from each of the above extraction steps may be pooled to provide the liquid extract for use in step (i).

Preferably, the process as described above, for the preparation of the liquid extract for use in step (i), is performed at a temperature below room temperature (i.e., below 25°C), such as at a temperature of from 1 to 10°C or from 2 to 7°C. The resulting supernatant is collected and, if it is to be stored, is preferably frozen in liquid nitrogen and stored at around -70°C.

Routine methods for the detection of the phospholipase D activity may be used to monitor the efficiency of the above extraction process to ensure that adequate quantities of phospholipase D are extracted from the source carrot.

Using the above extraction method, it is possible to obtain a high yield of liquid extract from the source carrot. For example, 0.6 to 0.67 litres of liquid extract may be extracted from 1kg of raw carrot

It will be appreciated that the liquid extract produced by the above extraction method may contain residues. For example, crude carrot extracts may contain floating lipids saturated with carotin. Hence, in the invention, the liquid extract is subjected to a clarification process prior to step (iii) to produce a clarified liquid extract. Therefore, the liquid extract is in the form of a clarified liquid extract.

The clarification process comprises contacting the liquid extract with a clarifying agent comprising one or more metal ions to produce a suspension comprising a clarified liquid extract and a precipitate. The resulting clarified liquid extract is then separated and collected from the suspension.

Thus, the process of the invention comprises the following steps prior to step (iii):
contacting the liquid extract of step (i) with a clarifying agent comprising one or more metal ions to produce a suspension comprising a clarified liquid extract and a precipitate; and
separating and collecting the clarified liquid extract from the suspension produced in step (ii).

Typically, the one or more metal ions in the clarifying agent are mono- or divalent ions, for example, ions of calcium, sodium, potassium, magnesium and mixtures thereof. For example, a halide salt of a suitable metal ion as described above may be used. Divalent calcium ions are preferred, which are typically provided in the form of calcium chloride.

The concentration of the one or more metal ions in step (ii) preferably ranges from 20 to 60 mM, such as from 30 to 50 mM or from 35 to 45 mM.

The contacting of the liquid extract of step (i) with a clarifying agent in step (ii) is preferably carried out at a temperature below room temperature, i.e., below 25°C, such as at a temperature of from 1 and 20°C, more preferably from 1 to 15°C, such as from 2 to 10°C or from 2 to 7°C. Upon contacting, the mixture is ideally mixed (e.g., by mechanical stirring).

Step (ii) is carried out at a pH in the range of from 7 to 9. The desired pH may be obtained using methods known in the art, for example by the addition of any suitable base, such as sodium hydroxide or ammonium hydroxide.

In a preferred embodiment of step (ii), after the liquid extract of step (i) and the clarifying agent have been contacted and the pH has been adjusted as desired, the temperature of the mixture is preferably dropped to around 0°C and the mixture left for a sufficient amount of time (for example, about 20 minutes) for the precipitate to form.

The clarified liquid extract produced in step (ii) may be separated from the precipitate using known methods in the art, such as by centrifugation and/or filtration. For example, the suspension may be filtered and the filtrate centrifuged at around 9000 rpm for about 15 minutes.

During separation from the suspension the temperature is preferably maintained below room temperature, such as at a temperature of from 1 and 20°C, more preferably from 1 to 15°C, such as from 2 to 10°C or from 2 to 7°C.

Typically, the clarification process result in minor reductions in the amount of phospholipase D in the liquid extract. For example, the clarification process produces less than 10 % by weight reduction in the amount of phospholipase D in the clarified liquid extract compared to the unclarified liquid extract.

The precipitating agent for use in the process of the invention is preferably in the form of an aqueous emulsion. The precipitating agent comprises phospholipids. The phospholipids may be derived from synthetic or natural sources. Representative examples of phospholipids that may be present in the second precipitating agent include phosphatidylcholine, phosphatidylethanolamine, phosphatidylinositol, phosphatidyl glycerol, sphingomyelin and mixtures thereof. The preferred phospholipid is phosphatidylcholine.

The precipitating agent used in the invention may be derived from naturally occurring substances obtained from animal and vegetable sources such as lecithin, cephalin, and sphingomyelin. Preferably the precipitating agent is derived from lecithin.

Lecithin contains a mixture of glycolipids, triglycerides, and phospholipids (e.g., phosphatidylcholine, phosphatidylethanolamine, and phosphatidylinositol) and may be derived from natural sources or synthetic sources. Preferably, lecithin for use in the preparation of the precipitating agent is obtained from animal or vegetable sources, such as from soy beans, egg yolks or rapeseed, using conventional processing methods. A suitable commercially available preparation of lecithin includes, for example, Membranol-35.

Preferably, the phospholipid content of lecithin for use in the preparation of the precipitating agent is greater than 15 % by weight, more preferably greater than 25 % by weight, such as greater than 30 % by weight or greater than 50 % by weight.

In a preferred embodiment, the amount by weight of phospholipids in the precipitating agent ranges from 0.1 to 7 % by weight, such as from 0.5 to 4 % by weight, more preferably from 0.8 to 3 % by weight, or from 0.8 to 2 % by weight.

The amount of phospholipids in step (iii), based on the total reaction volume, is preferably in the range of from 0.05 to 1 % by weight, more preferably from 0.1 to 0.5 % by weight, such as from 0.1 to 0.3 % by weight.

The phosphatidylcholine content, by weight of the phospholipids present in the precipitating agent, is preferably greater than 15 % by weight. For example, the phosphatidylcholine content may range from 20 to 100 % by weight, such as from 20 to 80 % by weight, from 25 to 60 % by weight, or from 25 to 40 % by weight of the phospholipids in the precipitating agent.

Optionally, the precipitating agent further comprises one or more metal ions. The one or more metal ions are preferably mono- or divalent ions, for example, ions of sodium, potassium, calcium, magnesium and mixtures thereof. For example, the precipitating agent may comprise an aqueous solution of a halide salt of a suitable metal ion. Divalent calcium ions are preferred, which are typically provided in the form of calcium chloride.

The concentration of the one or more metal ions in step (iii) preferably ranges from 20 to 60 mM, such as from 30 to 50 mM or from 35 to 45 mM.

In the preferred embodiment, in which the liquid extract is subjected to a clarification process, it will be appreciated that one or more metal ions will already be present in the clarified liquid extract and, as a consequence, it is not necessary for the precipitating agent to comprise one or more metal ions. However, due to the fact that some calcium ions may be bound to the precipitate from the clarification, in a preferred embodiment, the precipitating agent comprises one or more metal ions, preferably in an amount such that the concentration of the metal ions in step (iii) falls within the preferred concentration ranges as specified above.

The precipitating agent comprises at least one surfactant. Ionic or non-ionic surfactants are preferably present in the second precipitating agent, with ionic surfactants being particularly preferred. Suitable ionic surfactants include anionic surfactants, for example, a salt of a carboxylic acid, such as cholic acid, a salt of a (C₄ to C₂₄) linear alkyl sulphate, such as sodium dodecyl sulphate, or a mixture thereof. Suitable salts of cholic acid include, for example, food grade salts such as sodium salts. Sodium dodecyl sulphate is the preferred surfactant.

The amount of surfactant in the precipitating agent is preferably in the range of from 0.05 to 5 % by weight, more preferably from 0.1 to 3 % by weight or from 0.2 to 1.5% by weight

The amount of surfactant in step (iii), based on the total reaction volume, preferably ranges from 0.01 to 3 % by weight, more preferably from 0.05 to 1.0, most preferably from 0.08 to 0.16 % by weight.

In a preferred embodiment, the precipitating agent is prepared as an aqueous emulsion. Advantageously, the precipitating agent is prepared as a homogeneous mixture. This may be achieved, for example, simply by mechanically stirring the precipitating agent in an aqueous medium for a suitable amount of time. The presence of the surfactant may help to promote the dispersion of the components of the precipitating agent within the aqueous medium.

The precipitating agent preferably has a pH greater than 7, preferably a pH of from 7 to 9, such as from 7 to 8.5 or from 7.2 to 8. This pH can be achieved using known methods in the art, for example by the addition of any suitable base, such as sodium hydroxide or ammonium hydroxide.

Step (iii) of the process of the invention is preferably performed at a temperature below room temperature, i.e., at a temperature below 25°C. Most preferably, step (iii) is performed at a temperature of from 0 to 15°C, such as from 1 to 10°C or from 1 to 5°C. Typically, the pH of the mixture in step (iii) is greater than 7 (for example, at a pH in the range of from 7 to 9, most preferably, in the range of from 7 to 7.5).

Without wishing to be bound by theory, it is believed that during step (iii) of the invention, phospholipase D In the liquid extract is adsorbed, complexed or otherwise associated onto the precipitate formed from the one or more metal ions and the surfactant. For example, when the liquid extract is contacted with the precipitating agent comprising sodium dodecyl sulphate (SDS) in the presence of calcium chloride, it is believed that phospholipase D adsorbs onto the resulting calcium/SDS precipitate.

Step (ii) is preferably performed to the stage when at least 30 % by weight or at least 40 % by weight of the phospholipase D in the liquid extract is recovered in the precipitate. More preferably at least 50 % by weight, most preferably at least 60 % by weight or at least 66 % by weight of the phospholipase D in the liquid extract is recovered in the precipitate.

Although the rate of formation of the precipitation or recovery of the phospholipase D in the precipitate may vary depending on the temperature and other process variables, step (iii) is preferably carried out for a period of at least 5 minutes, more preferably for a period greater than 30 minutes or 1 hour. Most preferably, step (iii) is carried out for a period of from 30 minutes to 30 hours, such as from 1 to 24 hours, from 1 to 10 hours, from 1 to 5 hours or from 1.5 to 2.5 hours.

The precipitate produced in step (iii) may be separated from the supematant using known methods in the art, such as by centrifugation and/or filtration. For example, in step (iv) the suspension may be centrifuged at around 6000 rpm for about 15 minutes.

During step (iv) the temperature is preferably maintained below room temperature, such as in the range of from 1 and 20°C, more preferably from 1 to 15°C, such as from 2 to 10°C or from 2 to 7°C.

Typically, the collected precipitate is subsequently dried. Drying the precipitate may be carried out using known methods in the art. In a preferred embodiment, the precipitate is suspended in water, frozen (in liquid nitrogen, for example) and lyophilized.

In order to improve the shelf-life or preserve the activity of the phospholipase D produced by the invention, the phospholipase D is preferably lyophilized in the presence of potassium chloride, histidine-HCl, calcium chloride, sodium acetate or mixtures thereof, typically, at a pH of from 5 to 7. Most preferably, the phospholipase D produced by the invention is lyophilized in the presence of potassium chloride and sodium acetate at a pH of from 5 to 6.

The amount of phospholipase D produced, based on the total volume of liquid extract, is preferably greater than 0.2 % by weight, such as greater than 0.3 % by weight or greater than 0.5 % by weight, most preferably greater than 0.7 % by weight.

The phospholipase D produced by the process of the invention may be used in the preparation of phospholipids. Representative examples of phospholipids that may be produced using the phospholipase D include phosphatidylserine, phosphatidic acid, phosphatidylinositol, phosphatidylglycerol, phosphatidylethanol, phosphatidylglucose, phosphatidylbutanol, phosphatidylmethanol, phosphatidylethanolamine and mixtures thereof.

The following non-limiting examples illustrate the invention and do not limit its scope in any way. In the examples and throughout the specification, all percentages, parts and ratios are by weight unless indicated otherwise.

### Examples

### Example 1

### PLD preparation from carrot by Ca-SDS-Membranol-35 enzyme precipitation

3.9 kilograms of carrot were firstly chilled to 4°C, washed and chopped. A sap was obtained with juice extractor Braun MP 80 at 4°C. Additional PLD that remained in the hard wastes (residues) of the carrot was recovered by re-processing the hard wastes in the juice extractor. Altogether 2400 ml carrot juice and 1500 g wastes were received. The juice was filtered through 4 layers of gauze. The filtrate was centrifuged at 9000 rpm (Beckman J-21 centrifuge, JA-14 rotor) for 15 min at 4°C. After centrifugation 2300 ml supernatant was collected and frozen in liquid nitrogen and stored at -70°C. The supernatant was bright yellow.

Carrot juice was quickly defrosted with lukewarm water, and then placed in an ice water bath. To 300 ml of defrosted juice, 14 ml of 1 M CaCl₂ was slowly added under constant mixing up to final concentration of 40 mM. The pH of the mixture was then carefully adjusted to pH 7.4 by adding of 1 M NH₄OH and exposed for 20 min at 0°C. The formed precipitate was separated by centrifugation at 6000 rpm (Beckman J-21 centrifuge, JA-14 rotor) for 15 min at 4°C. The supernatant (clarified juice) was collected.

To 160 ml clarified juice, 16 ml SDS-Membranol-35 emulsion (see Example 2 for the preparation of the emulsion) was added and the mixture was well mixed. 8 ml of 1M CaCl₂ was added in small portions (1 ml). Under stirring 5 ml 1N NH₄OH solution was added dropwise until a pH of about 7.15 was reached. The whole mixture was kept in an ice bath for approximately 2 hours. The formed precipitate was separated by centrifugation at 6000 rpm (Beckman J-21 centrifuge, JA-14 rotor) for 15 min at 4°C. The pellet was suspended in about 5 ml of water, frozen in liquid nitrogen and lyophilized.

After lyophilization 1.29 g lyophilized preparation was received. From 1 ml carrot juice approximately 8 mg powder was obtained (see activity in Table 1)

### Example 2

### Preparation of SDS-Membranol-35 emulsion

To 1.2 ml Membranol-35 (Lipid Nutrition B.V., Wormerveer, The Netherlands), 0.15 ml 1N NaOH and 2.4 ml 10% water solution of sodium dodecyl sulfate (SDS) was added while stirring. Then water in small portions (5 times x 1 ml) was added under vigorous stirring until homogeneous mixture was formed. Then water was added to 40 ml total and the mixture was stirred for about 30 min on a shaker.

### Example 3

### PLD preparation from carrot by ethanol precipitation

To one volume of clarified and chilled (0°C) juice, 0.4 volume of chilled ethanol was slowly added under constant mixing and mixture temperature controlled in a range 0°C to -5°C. The mixture was exposed for 30-60 min at -13°C -15°C, then centrifuged at -10°C at 9000 rpm (JA-14 rotor, 15 min). The supernatant was discarded and the precipitate was quickly suspended in 0.1-0.2 volume (of the initial juice volume) of water with constant torque direct-drive laboratory mixer and the suspension was centrifuged at 14,000 rpm for 15 min in JA-20 rotor at 2-4°C to give supernatant 1. Extraction with water was repeated one more time to give supernatant 2. Both supernatants (1 and 2) were combined and 2 M KCI, 1 M CaCl₂ and 2 M potassium acetate (pH 5.6) were added to give final concentrations 220 mM, 40 mM and 20 mM, respectively (see activity in Table 1)

**Table 1**

| **Characteristics of PLD preparations of the ethanol and Ca-SDS-Membranol-35 precipitated PLD (determined by pH-titrimetry and/or TLC).** | | |
|---|---|---|
| **PLD preparation** | **Specific activity of PLD related to 1 ml of carrot juice (µmol/min.ml)** | **Specific activity of PLD related to 1 mg of preparation (µmol/min.mg)** |
| * Carrot juice obtained | 10.69 ± 0.5 | About 0.01 |
| µ Vacuum-dried Ca-SDS-Membranol-35 preparation precipitated from clarified carrot juice | 5.3 ± 0.33 | 0.66 ± 0,04 |
| ^{µ} Vacuum-dried PLD precipitated from clarified carrot juice with ethanol in presence of sodium acetate | 0.62 ± 0.08 | 0.31 ± 0.04 |

| | | |
|---|---|---|
| * The activity was determined by titrimetry ^{µ} The activity was determined by visually comparing the intensity of the PA spots on the TLC plate. Ranges of 4 doubly diluted specimens of the standard PLD preparation and examined enzyme preparations were compared. Activity of the standard PLD preparation was determined by titrimetry. | | |

### Example 4

### Effect of pH on the Extraction

Freshly extracted carrot juice was centrifuged at 12000 rpm (Beckman J-21 centrifuge, JA-14 rotor) for 60 min at 4°C and supernatant (S) was collected. To 50 ml of the supernatant, 1 ml of 2 M CaCl₂ solution was added (to form SI). The pH of this mixture was neutralized by the addition of 3M NH₄OH solution, 1 ml aliquots were picked up at different pH and centrifuged for 4 minutes at 5000 rpm at room temperature to obtain SII. The PLD activity of SII samples was analyzed by pH-metry. of SI. The results are summarized in the table below.

### Protein determination

The absorption data of the protein solutions were obtained at UV wavelengths 280 nm and 260 nm (preliminary aliquots of these solutions were picked up to get optical density about 0,1-0,5). Then using absorption data and Practical Course of Biochemistry, S.E Severin and G.A. Solovyova eds., M., MSU, 1989, *in Russian,* the corresponding protein concentrations in aliquots were determined.

### PLD activity determination: pH titrimetry

PLD activity was monitored with digital pH meter. Aquiline 410 was calibrated with pH standard solutions pH 4.01, 6.86 and 9.26 at room temperature. Reaction mixture for titrimetry analysis of the PLD activity contained CaCl₂ and Membranol/SDS emulsion, pH - 7,0 (final concentrations: Membranol 6 mg/ml, SDS - 0.18%, 10 mM CaCl₂).

CaCl₂/pH-clarification of SI: pH at the constant CaCl₂ concentration on the PLD activity

| Sample | Final pH | Total protein (%) in SII | Specific PLD activity × 10⁻², µmol/sec·mg | Purification factor |
|---|---|---|---|---|
| 1 | 6.14 | 85 | 3.06 | 1.25 |
| 2 | 6.46 | 82.5 | nd | nd |
| 3 | 6.78 | 50 | nd | nd |
| 4 | 7.00 | 42.5 | nd | nd |
| 5 | 7.18 | 42.5 | 4.47 | 1.82 |
| 6 | 7.41 | 42,5 | 4.35 | 1.78 |
| 7 | 7.61 | 40 | 5.38 | 2.2 |
| 8 | 7.95 | 37.5 | 6.13 | 2.5 |
| 9 | 8.48 | 37.5 | 6.1 | 2.49 |
| 10 | 8.91 | 35 | 6.5 | 2.1 |

### Example 5

### Effect of surfactant

### Precipitation of PLD with SDS-Lecithin emulsion

### Preparation of SDS-Lecithin-emulsion

To 2.4 g of sunflower Lecithin (Lipid Nutrition B.V., Wormerveer, The Netherlands), 0.15 ml 1N NaOH and 2.4 ml 10% water solution of sodium dodecyl sulphate (SDS) was added while stirring. Then water in small portions (5 x 1 ml) was added under vigorous stirring until a homogeneous mixture was formed. Then water was added to 40 ml total and the mixture was stirred for about 30 min on a shaker.

### Precipitation of PLD with SDS-Lecithin-emulsion

To 16 ml of clarified carrot juice, 1.6 ml SDS-Lecithin-emulsion was added and the mixture was well mixed. Under stirring a few drops of 3N NH₄OH solution were added drop wise until a pH of 7.14 was reached. The whole mixture was kept in an ice bath for approximately 1 hour. The formed precipitate was separated by centrifugation at 3500 rpm (Jouan-Paris K-63F) for 10 min at 4°C. The pellet was suspended in about 0.5 ml of water, frozen in liquid nitrogen and lyophilized.

After lyophilization 150 mg lyophilized preparation was received. From 1 ml carrot juice approximately 9.3 mg of powder was obtained with the specific activity about 2.1x10⁻² µmol per minute per 1 mg of preparation.

### Precipitation of PLD without SDS with lecithin-emulsion

### Preparation of Lecithin-emulsion

To 2.4 g of sunflower Lecithin (Lipid Nutrition B.V., Wormerveer, The Netherlands), 0.15 ml 1N NaOH was added. Then water in small portions (5 x 1 ml) was added under vigorous stirring until a homogenous mixture was formed. Then water was added to 40 ml total and the mixture was stirred for about 30 min on a shaker.

### Precipitation of PLD without SDS with lecithin-emulsion

To 16 ml of clarified juice, 1.6 ml Lecithin emulsion was added and the mixture was well mixed. Under stirring a few drops of 3N NH₄OH solution were added until a pH 7.14 was reached. The whole mixture was kept in an ice bath for approximately 1 hour. The formed precipitate was separated by centrifugation at 3500 rpm (Jouan-Paris K-63F) for 10 min at 4°C. The pellet was suspended in about 0.5 ml of water, frozen in liquid nitrogen and lyophilized.

After lyophilization 172 mg lyophilized preparation was received. From 1 ml carrot juice approximately 10.7 mg of powder was obtained with the specific activity about 1.0x10⁻² µmol per minute per 1 mg of preparation.

### Example 6

### Process without Lecithin

### Precipitation of PLD without lecithin by addition of CaCl₂, then SDS

To 2.4 g of 10 % water solution of sodium dodecyl sulphate (SDS) 0.15 ml 1 N NaOH were added while stirring. Then water was added to 40 ml total to make an SDS-solution.

To 16 ml of clarified juice, 0.8 ml 1 M CaCl₂ was added dropwise then 1.6 ml of the SDS-solution was added and the mixture was well mixed. Under stirring a few drops of 1 N NH₄OH solution were added drop wise until a pH 7.16 was reached. The whole mixture was kept in an ice bath for approximately 1 hour. The formed precipitate was separated by centrifugation at 3500 rpm (Jouan-Paris K-63F) for 10 min at 4°C. The pellet was suspended in about 0.5 ml of water, frozen in liquid nitrogen and lyophilized.

After lyophilization 17.2 mg lyophilized preparation was received. From 1 ml carrot juice approximately 1.1 mg of powder was obtained with the specific activity about 48.9x10⁻² µmol per minute per 1 mg of preparation.

### Example 7

### Effect of Different Surfactants

The extraction of PLD was carried out substantially as described above using a range of difficult surfactants.

To 0.2 g of sunflower Lecithin (Lipid Nutrition B.V., Wormerveer, The Netherlands), 0.04 ml 1N NaOH and 0.6 ml of 10 % water solution of the surfactant was added. Then water in portions (2 x 4 ml) was added under vigorous stirring until a homogenous mixture was formed. Then water was added to 10 ml total and the mixture was stirred for about 30 min on a shaker.

To 16 ml of clarified juice, 1.6 ml of the above Lecithin emulsion was added and the mixture was well mixed, then 0.8 ml of 1 M CaCl₂ was added dropwise. Under stirring a few drops of 3N NH₄OH solution were added until a pH 7.17 was reached. The whole mixture was kept in an ice bath for approximately 2 hours. The formed precipitate was separated by centrifugation of 17.4 ml of the mixture at 3500 rpm (Jouan-Paris K-63F) for 10 min at 4°C. The pellet was suspended in about 0.5 ml of water, frozen in liquid nitrogen and lyophilized. For the activity measurement, the pellet from 1 ml of the mixture was collected by centrifugation at 4,000 or 12,000 rpm on the Beckman bench centrifuge for 5 minutes.

The results are set out in the following table.

| Surfactant | [Ca] added mM | Pellet weight mg | Activity |
|---|---|---|---|
| | | | 10⁻² µmol per minute per 1 ml of mixture |
| Non-ionic surfactant | | | |
| Triton X-100 | 42 | 22 | 21 |
| Brig 35 | 42 | 20 | 17 |
| Brig 58 | 42 | 12 | 10 |
| Cationic surfactant | | | |
| CTAB | 42 | 100 | 36 |
| CTAB (no CaCl₂ added) | - | 38 | 9 |
| Zwitterionic surfactant containinq sulfonyl-group | | | |
| Zwittergent 3-06 (MW 251.6) | 42 | 96 | 70 |
| Zwittergent 3-06 (MW 279.6) | 42 | 80 | 67 |
| Zwittergent 3-08 (MW 335.6) | 42 | 100 | 42 |
| Zwittergent 3-16 (MW 391.6) | 42 | 32 | 43 |
| Anionic surfactant | | | |
| Sarcosyl | 42 | 87 | 70 |
| SDS | 42 | 93 | 161 |

### Example 8

### Effect of surfactant concentration

To 0.96 g of sunflower lecithin (Lipid Nutrition B.V., Wormerveer, The Netherlands), 0.15 ml 1N NaOH (and varying amounts of 10% water solution of sodium dodecyl sulphate (SDS)) was added while stirring. Then water in small portions (5 time x 1 ml) was added under vigorous stirring until a homogenous mixture was formed. Then water was added to 40 ml total and the mixture was stirred for about 30 min on a shaker.

To 16 ml of clarified juice, 1.6 ml SDS-Lecithin-emulsion was added and the mixture was well mixed, then 0,8 ml of 1 M CaCl₂ solution was added dropwise. Under stirring a few drops of 3N NH₄OH solution were added dropwise until a pH of 7.18 was reached. The whole mixture was kept in an ice bath for approximately 2 h. The formed precipitate was separated by centrifugation at 3500 rpm (Jouan-Paris K-63F) for 10 min at 4°C. The pellet was suspended in about 0.5 ml of water, frozen in liquid nitrogen and lyophilized.

Corresponding experiments were carried out using 1.2 g of Soya Membranol-35 (Lipid Nutrition B.V., Wormerveer, The Netherlands) in 15 ml 1N NaOH with varying amounts of SDS, in place of the sunflower lecithin.

The results of the experiments are set out in the following table.

| Phospholipid-emulsion | Final SDS concentration in emulsion, % | Amount of PLD preparation, mg/ 1 ml SI | Activity, 10⁻² µmol per minute per 1 mq of preparation |
|---|---|---|---|
| Lecithin- SDS | 0 | 3.9 | 4.7 |
| Lecithin- SDS | 0.6 | 5.6 | 22.0 |
| Lecithin- SDS | 0.2 | 5.4 | 5.2 |
| Lecithin- SDS | 3 | 5.1 | 12.0 |
| Membranol- SDS | 0 | 5.4 | 2.7 |
| Membranol- SDS | 0.6 | 5.2 | 26.7 |
| Membranol- SDS | 0.2 | 3.7 | 4.0 |
| Membranol- SDS | 3 | 8.4 | 6.3 |

### Example 9

### Different Sources of Lecithin

Experiments were carried out as described above in Example 5, using sunflower oil or different lecithins in an amount of 1.2 g to replace the sunflower lecithin used in that example.

The results were as follows:

| Emulsion | SDS | CaCl₂ added, 42 mM | Mg(CH₃ COO)₂ added, 21 mM | Weight of pellet | Activity |
|---|---|---|---|---|---|
| | | | | | 10⁻² µmol per minute per 1 ml of mixture |
| Sunflower oil** | 0 | + | | 25 | 27 |
| SDS/ Sunflower oil** | 0.2 | + | | 7 | 25 |
| SDS/ Sunflower oil** | 0.6 | + | | nd | 23 |
| Sunflower oil** | 0 | + | | 50 | 10 |
| Membranol oil** | 0 | + | | 54 | 13 |
| SDS/ Membranol oil** | 0.6 | + | | 144 | 58 |
| SDS/ Membranol oil** | 0.6 | | + | 69 | 49 |
| De-oiled lecithin | 0 | + | | 72 | 17 |
| SDS/ De-oiled lecithin | 0.6 | + | | 88 | 102 |
| SDS/ De-oiled lecithin | 0.6 | | + | 77 | 119 |
| Egg yolk extract | 0 | + | | 35 | 14 |
| Egg yolk extract | 0 | | + | - | 18 |
| SDS/Egg yolk extract | 0.6 | + | | 102 | 79 |

## Claims

1. A process for the preparation of a phospholipase D from carrot comprising the following steps:
(i) providing a liquid carrot extract;
(ii) contacting the liquid extract of (i) with a clarifying agent comprising one or more metal ions to produce a suspension comprising a clarified liquid extract and a precipitate;
(iii) combining:
the clarified liquid extract of (ii) with a precipitating agent comprising at least one surfactant in admixture with phospholipids and optionally mono-, di- or tri-glycerides or a mixture thereof to produce a suspension comprising a supernatant and a precipitate; and
(iv) separating and collecting the precipitate from the suspension produced in (iii),
wherein step (ii) is carried out at a pH of from 7 to 9
and wherein the process comprises separating and collecting the clarified liquid extract from the suspension produced in step (ii).

2. A process as claimed in Claim 1, wherein the precipitating agent is in the form of an aqueous emulsion.

3. A process as claimed in any one of the preceding claims, wherein the precipitating agent is derived from lecithin.

4. A process as claimed in any one of the preceding claims, wherein the precipitating agent of (iii) further comprises one or more metal ions.

5. A process as claimed in any one of the preceding claims, wherein the surfactant is an anionic surfactant.

6. A process as claimed in Claim 5, wherein the surfactant is selected from salts of cholic acid, sodium dodecyl sulphate and mixtures thereof.

7. A process as claimed in Claim 6, wherein the salt of cholic acid is sodium cholate.

8. A process as claimed in any one of the proceeding claims, wherein the one or more metal ions are divalent calcium ions.

9. A process as claimed in Claim 8, wherein the divalent calcium ions are provided in the form of calcium chloride.

10. A process according to any one of the preceding claims, wherein step (iii) is carried out at a pH of from 7 to 9.

11. A process according to any one of the preceding claims, wherein step (iii) of the process is performed at a temperature below 25°C, for example from 0 to 15°C.

12. A process according to any one of the preceding claims, wherein the collected precipitate is subsequently dried.

13. A process according to any one of the preceding claims, wherein the amount of surfactant is in the range of from 0.05 to 3% by weight, or lecithin for use in the preparation of the precipitating agent is obtained from animal or vegetable sources.

## Patentansprüche

1. Verfahren zur Herstellung einer Phospholipase D aus Karotten, umfassend die folgenden Stufen:
(i) Bereitstellen eines flüssigen Karottenextrakts;
(ii) Inkontaktbringen des flüssigen Extrakts von (i) mit einem Klärungsmittel, das ein oder mehrere Metallionen umfasst, unter Bildung einer Suspension, die einen geklärten flüssigen Extrakt und einen Niederschlag umfasst;
(iii) Kombinieren des geklärten flüssigen Extrakts von (ii) mit einem Fällungsmittel, das mindestens ein grenzflächenaktives Mittel in einem Gemisch mit Phospholipiden und optional Mono-, Di- oder Triglyceriden oder einem Gemisch derselben umfasst, unter Bildung einer Suspension, die einen Überstand und einen Niederschlag umfasst; und
(iv) Abtrennen und Gewinnen des Niederschlags aus der in (iii) gebildeten Suspension,
wobei die Stufe (ii) bei einem pH-Wert von 7 bis 9 durchgeführt wird
und wobei das Verfahren das Abtrennen und Gewinnen des geklärten flüssigen Extrakts aus der in Stufe (ii) gebildeten Suspension umfasst.

2. Verfahren nach Anspruch 1, wobei das Fällungsmittel in der Form einer wässrigen Emulsion vorliegt.

3. Verfahren nach einem der vorhergehenden Ansprüche, wobei das Fällungsmittel von Lecithin abgeleitet ist.

4. Verfahren nach einem der vorhergehenden Ansprüche, wobei das Fällungsmittel von (iii) ferner ein oder mehrere Metallionen umfasst.

5. Verfahren nach einem der vorhergehenden Ansprüche, wobei das grenzflächenaktive Mittel ein anionisches grenzflächenaktives Mittel ist.

6. Verfahren nach Anspruch 5, wobei das grenzflächenaktive Mittel aus Cholsäuresalzen, Natriumdodecylsulfat und Gemischen derselben ausgewählt ist.

7. Verfahren nach Anspruch 6, wobei das Cholsäuresalz Natriumcholat ist.

8. Verfahren nach einem der vorhergehenden Ansprüche, wobei das eine oder die mehreren Metallionen zweiwertige Calciumionen sind.

9. Verfahren nach Anspruch 8, wobei die zweiwertigen Calciumionen in der Form von Calciumchlorid bereitgestellt werden.

10. Verfahren nach einem der vorhergehenden Ansprüche, wobei die Stufe (iii) bei einem pH-Wert von 7 bis 9 durchgeführt wird.

11. Verfahren nach einem der vorhergehenden Ansprüche, wobei die Stufe (iii) des Verfahrens bei einer Temperatur von unter 25°C, beispielsweise von 0 bis 15°C, durchgeführt wird.

12. Verfahren nach einem der vorhergehenden Ansprüche, wobei der gewonnene Niederschlag anschließend getrocknet wird.

13. Verfahren nach einem der vorhergehenden Ansprüche, wobei die Menge des grenzflächenaktiven Mittels im Bereich von 0,05 bis 3 Gew.-% liegt oder das Lecithin zur Verwendung bei der Herstellung des Fällungsmittels aus tierischen oder pflanzlichen Quellen erhalten wird.

## Revendications

1. Procédé de préparation d'une phospholipase D à partir de carotte comprenant les étapes suivantes :
(i) fournir un extrait de carotte liquide ;
(ii) mettre en contact l'extrait liquide de (i) avec un agent de clarification comprenant un ou plusieurs ions métalliques pour produire une suspension comprenant un extrait liquide clarifié et un précipité ;
(iii) combiner :
l'extrait liquide clarifié de (ii) avec un agent de précipitation comprenant au moins un agent tensioactif en mélange avec des phospholipides et facultativement des mono-, di- ou tri-glycérides ou l'un de leurs mélanges pour produire une suspension comprenant un surnageant et un précipité ; et
(iv) séparer et collecter le précipité de la suspension produite dans (iii),
dans lequel l'étape (ii) est réalisée à un pH de 7 à 9
et dans lequel le procédé comprend la séparation et la collecte de l'extrait liquide clarifié de la suspension produite à l'étape (ii).

2. Procédé selon la revendication 1, dans lequel l'agent de précipitation se présente sous la forme d'une émulsion aqueuse.

3. Procédé selon l'une quelconque des revendications précédentes, dans lequel l'agent de précipitation est dérivé de la lécithine.

4. Procédé selon l'une quelconque des revendications précédentes, dans lequel l'agent de précipitation de (iii) comprend en outre un ou plusieurs ions métalliques.

5. Procédé selon l'une quelconque des revendications précédentes, dans lequel l'agent tensioactif est un agent tensioactif anionique.

6. Procédé selon la revendication 5, dans lequel l'agent tensioactif est choisi parmi les sels d'acide cholique, le dodécylsulfate de sodium et leurs mélanges.

7. Procédé selon la revendication 6, dans lequel le sel d'acide cholique est le cholate de sodium.

8. Procédé selon l'une quelconque des revendications précédentes, dans lequel le(s) un ou plusieurs ions métalliques sont des ions calcium divalents.

9. Procédé selon la revendication 8, dans lequel les ions calcium divalents sont fournis sous la forme de chlorure de calcium.

10. Procédé selon l'une quelconque des revendications précédentes, dans lequel l'étape (iii) est réalisée à un pH de 7 à 9.

11. Procédé selon l'une quelconque des revendications précédentes, dans lequel l'étape (iii) du procédé est réalisée à une température en dessous de 25 °C, par exemple de 0 à 15 °C.

12. Procédé selon l'une quelconque des revendications précédentes, dans lequel le précipité collecté est ultérieurement séché.

13. Procédé selon l'une quelconque des revendications précédentes, dans lequel la quantité d'agent tensioactif est dans la plage de 0,05 à 3 % en poids, ou bien la lécithine à utiliser dans la préparation de l'agent de précipitation est obtenue à partir de sources animales ou végétales.
